(19)

Europäisches
Patentamt
European
Patent Office
Office européen
des brevets

(11) **EP 4 667 888 A1**

(12) **EUROPEAN PATENT APPLICATION**

(43) Date of publication:
24.12.2025 Bulletin 2025/52

(21) Application number: 25181798.7

(22) Date of filing: **10.06.2025**

(51) International Patent Classification (IPC):
*G01M 3/38* (2006.01)     *G01N 21/3504* (2014.01)

(52) Cooperative Patent Classification (CPC):
**G01M 3/38; G01N 21/3504**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **20.06.2024  JP 2024099932**

(71) Applicant: **Konica Minolta, Inc.
Tokyo 100-7015 (JP)**

(72) Inventors:
• **ISHIKAWA, Tetsuya**
  **Tokyo, 100-7015 (JP)**
• **AMANO, Suguru**
  **Tokyo, 100-7015 (JP)**

(74) Representative: **Gille Hrabal
Partnerschaftsgesellschaft mbB
Patentanwälte
Brucknerstraße 20
40593 Düsseldorf (DE)**

(54) **GAS FLOW RATE ESTIMATION DEVICE, GAS FLOW RATE ESTIMATION METHOD, GAS FLOW RATE ESTIMATION PROGRAM, LEAKAGE GAS DETECTION DEVICE, LEAKAGE GAS DETECTION METHOD, AND DETECTION DATA PROCESSING DEVICE**

(57)     A gas flow rate estimation device (10) includes a map acquisition section (34) acquiring, from position information of a camera (20) capturing an image including information of an infrared ray, map information around the camera (20), a distance calculation section (37) calculating, based on the map information, a distance from the camera (20) to a gas cloud, a first calculation section (13) calculating, using time-series images, a gas velocity of the gas cloud and a gas passage time for which gas passes through a gas region, a second calculation section (15) calculating a gas concentration thickness product of the gas region by using image data of the gas region and a gas amount of the gas region by using the gas concentration thickness product and a distance calculated by the distance calculation section (37), and a third calculation section (16) calculating a flow rate estimation value of gas by using the gas passage time and the gas amount.

**FIG. 1**

EP 4 667 888 A1

## Description

BACKGROUND OF THE INVENTION

Technical Field

The present invention relates to an apparatus, a method, and a program for estimating a gas flow rate, an apparatus and a method for detecting a leakage gas, and a data processing apparatus for leakage gas detection.

Description of Related Art

A leakage gas detection device is known which detects a leaked gas from an image captured by a camera using infrared absorption of a specific wavelength by a gas. Such a leakage gas detection device can inspect gas leakage from a gas facility or the like from a remote place. In addition, a leakage gas detection device capable of estimating not only the presence or absence of leaked gas but also a gas flow rate has also been developed. For example, International Publication WO 2020-110411 describes a system including an infrared camera and a gas flow rate estimation device. The gas flow rate is a volume of gas flowing in a fixed time.

SUMMARY OF THE INVENTION

One of parameters greatly involved in the accuracy of flow rate estimation is distance information between an imaging position and a measurement target. The distance information is a distance in the depth direction. The distance information is used in calculation of the concentration thickness product. Conventionally, a value measured by a user with a laser range finder or the like is used as distance information. In the case of flow rate estimation, distance information needs to be measured and stored at the time of measurement. In the case of measurement without flow rate estimation, the distance information is not required. However, it may not be known whether the flow rate estimation is performed from the captured image data. Even in that case, it is necessary to measure and store the distance information every time, which increases the work burden on the user. On the other hand, in data in which distance information is not stored, flow rate estimation cannot be performed later. The present invention has been devised in order to solve such problems.

It is an object to provide a leakage gas detection device, a leakage gas detection method, and the like that do not require distance measurement by a user at the time of capturing an infrared image and are capable of estimating a flow rate with high accuracy.

In order to solve such a problem, a gas flow rate estimation device, a gas flow rate estimation method, a gas flow rate estimation program, a leakage gas detection device, a leakage gas detection method, and a detection data processing device according to the present invention have the following configurations (1) to (3).

(1) To achieve at least one of the abovementioned objects, according to an aspect of the present invention, a gas flow rate estimation device reflecting one aspect of the present invention includes a map acquisition section that acquires, from position information of a camera capturing an image including information of an infrared ray, map information around the camera, a distance calculation section that calculates, based on the map information, a distance from the camera to a gas cloud captured by the camera, a first calculation section that calculates, by using time-series images of the gas cloud captured by the camera, a gas velocity of the gas cloud and a gas passage time for which gas passes through a gas region extracted from the time-series images, a second calculation section that calculates a gas concentration thickness product of the gas region by using image data of the gas region included in the time-series images and that calculates a gas amount of the gas region by using the gas concentration thickness product and a distance calculated by the distance calculation section, and a third calculation section that calculates a flow rate estimation value of gas by using the gas passage time and the gas amount.

(2) To achieve at least one of the abovementioned objects, according to an aspect of the present invention, a gas flow rate estimation method reflecting one aspect of the present invention includes acquiring map information around a camera capturing an infrared image of a gas cloud from position information of the camera, calculating a distance from the camera to the gas cloud based on the map information, calculating, by using time-series images of the gas cloud captured by the camera, a gas velocity of the gas cloud and a gas passage time for which gas passes through a gas region extracted from the time-series images, calculating a gas concentration thickness product of the gas region by using image data of the gas region included in the infrared image and a gas amount of the gas region by using the gas concentration thickness product and a distance to the gas cloud, and calculating a flow rate estimation value of gas by using the gas passage time and the gas amount.

(3) To achieve at least one of the abovementioned objects, according to an aspect of the present invention, a gas flow rate estimation program reflecting one aspect of the present invention causes a computer to perform acquiring map information around a camera capturing an infrared image of a gas cloud from position information of the camera, calculating a distance from the camera to the gas cloud based on the map information, calculating, by using time-series images of the gas cloud captured by the camera, a gas velocity of the gas cloud and a gas passage time for which gas passes through a gas region extracted from the time-series images, calculating a gas concentration thickness product of the gas region by using image data of the gas region included in the time-series images and a gas amount of the gas region by using the gas concentration thickness product and a distance to the gas cloud, calculating a flow rate estimation value of gas by using the gas passage time and the gas amount.

## BRIEF DESCRIPTION OF THE DRAWINGS

The advantages and features provided by one or more embodiments of the invention will become more fully understood from the detailed description given hereinbelow and the appended drawings which are given by way of illustration only, and thus are not intended as a definition of the limits of the present invention:

Fig. 1 is a perspective view illustrating an outline of a leakage gas detection device according to an embodiment.
Fig. 2 is a block diagram illustrateing a hardware configuration of a leakage gas inspection device according to an embodiment.
Fig. 3 is a block diagram illustrating a functional configuration of the leakage gas inspection device according to an embodiment.
Fig. 4 shows a screen illustrating an image of a display part displayed at the time of imaging.
Fig. 5A shows a screen illustrating an extracted gas region.
Fig. 5B illustrates a screen for schematically explaining pixels.
Fig. 6 shows a screen illustrating distance calculation processing in the leakage gas inspection device according to an embodiment.
Fig. 7 is a side view illustrating a positional relationship between a camera and a gas cloud.
Fig. 8 is a perspective view. In the leakage gas detection device according to an embodiment, the camera and the gas flow rate estimation device may be separate bodies. A schematic illustration of the leakage gas detection device in such a case is Fig. 8.
Fig. 9A shows a screen illustrating map information displayed on a screen of a display part.
Fig. 9B shows a screen illustrating a distance calculation process in a modification example.
Fig. 10 is a block diagram illustrating a functional configuration of a detection data processing device according to an embodiment.
Fig. 11 is a flowchart of the leakage gas detection method according to an embodiment.
Fig. 12 is a flowchart illustrating flow rate calculation processing.
Fig. 13 is a flowchart of the leakage gas detection method according to an embodiment.
Fig. 14A is a flowchart of a data acquisition process in the leakage gas detection method according to an embodiment.
Fig. 14B is a flowchart of a data reading process in the leakage gas detection method according to an embodiment.
Fig. 15A is a flowchart of a data acquisition process in the leakage gas detection method according to an embodiment.
Fig. 15B is a flowchart of a data reading process in the leakage gas detection method according to an embodiment.

## DETAILED DESCRIPTION

Hereinafter, one or more embodiments of the present invention will be described with reference to the drawings. However, the scope of the invention is not limited to the disclosed embodiments.

Embodiments and modification examples of the present invention are described with reference to the drawings. Note that the present invention is not limited to these embodiments and modification examples.

Leakage Gas Detection Device

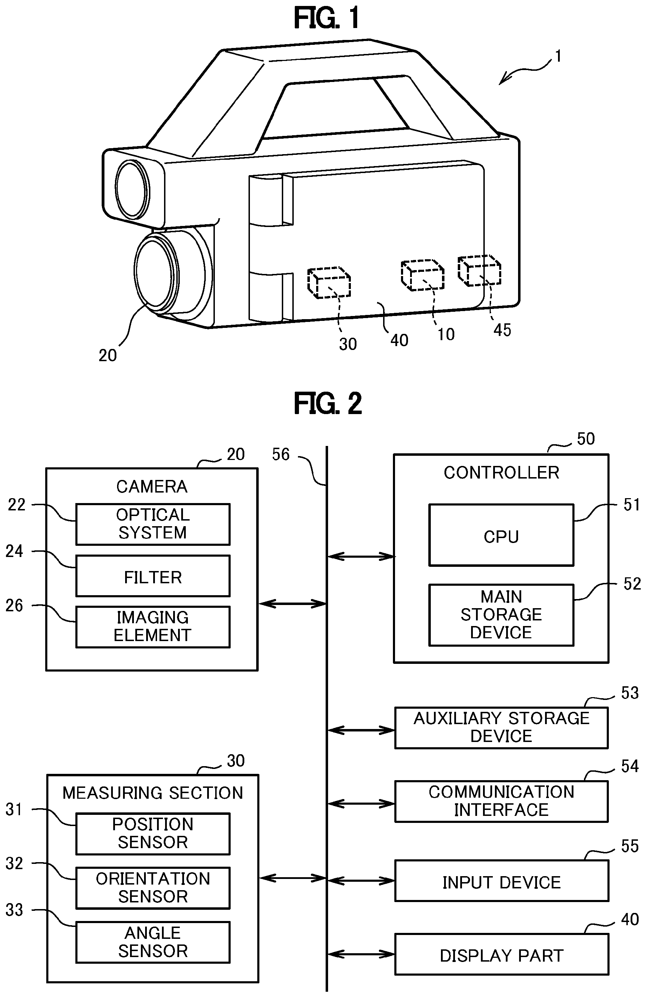

A leakage gas detection device 1 according to a first embodiment is described with reference to Figs. 1 to 8. The leakage gas detection device 1 detects leakage gas from a gas accommodation facility or the like. The inspection target gas is, for example, a hydrocarbon-based gas such as methane, ethane, or propane. In the case of a gas leak or the like, the gas is like a cloud in the vicinity of the place. The leakage gas detection device 1 can visualize and observe such a gas cloud on a screen. As illustrated in Fig. 1, the leakage gas detection device 1 includes a camera 20, a measuring section 30, a display part 40, and a gas flow rate estimation device 10. Here, the leakage gas detection device 1 further includes an acquired data storage section 45. Note that the display part 40 is folded in Fig. 1. Hereinafter, each component of the leakage gas detection device 1 is described.

Fig. 2 is a block diagram illustrating a hardware configuration of the leakage gas detection device 1. The leakage gas detection device 1 includes a camera 20, a measuring section 30, a controller 50, an auxiliary storage device 53, a communication interface 54, an input device 55, and a display part 40. The camera 20, the measuring section 30, the controller 50, the auxiliary storage device 53, the communication interface 54, the input device 55, and the display part 40 are connected to each other by a bus 56. Here, the gas flow rate estimation device 10 is incorporated as a function of the controller 50. Note that the gas flow rate estimation device 10 may be configured as independent hardware. The controller 50 is a device. The controller 50 controls the leakage gas detection device 1. As illustrated in Fig. 2, the controller 50 includes a central processing unit (CPU) 51 and a main storage device 52. The controller 50 performs information processing related to control of the leakage gas detection device 1. The main storage device 52 is a ROM or a RAM. The auxiliary storage device 53 is a hard disk or the like. The communication interface 54 is a connection device with a local area network (LAN) or the Internet. The input device 55 is a touch screen, a keyboard, or the like. The display part 40 is a display or the like. The storage device of the acquired data storage section 45 is included in the auxiliary storage device 53.

Camera

The camera 20 is an apparatus. The camera 20 captures an image of the leaking gas. The camera 20 can capture an infrared image. The infrared image is an image including information of an infrared ray. The camera 20 can capture time-series images. As illustrated in Fig. 2, the camera 20 includes an optical system 22, a filter 24, and an imaging element 26. The optical system 22 is a member that refracts light. The optical system 22 forms an image of a subject on the imaging element 26. The filter 24 is a member. The filter 24 allows an infrared ray having a specific wavelength to pass therethrough. The filter 24 is disposed between the optical system 22 and the imaging element 26. The filter 24 allows only an infrared ray having a specific wavelength to pass therethrough, out of light that has passed through the optical system 22. The wavelength to be passed varies depending on the type of gas. For example, when the detection target gas is methane, the filter 24 is a band-pass filter that transmits light having a wavelength of 3.2 $\mu$m to 3.4 $\mu$m. The imaging element 26 may be, for example, an indium antimonide (InSb) image sensor.

Measuring Section 30

The measuring section 30 is an apparatus. The measuring section 30 measures the position and the posture of the camera 20. The measuring section 30 is integrated with the camera 20. The measuring section 30 includes a position sensor 31, a orientation sensor 32, and an angle sensor 33. The position sensor 31 is a device. The position sensor 31 receives a signal from a satellite and acquires position information. The position sensor 31, for example, can use a satellite positioning system such as a global positioning system (GPS).

The orientation sensor 32 is an apparatus. The orientation sensor 32 acquires the orientations of north, south, east, and west among the posture information of the camera 20. An imaging orientation, that is, an orientation in which the camera 20 faces on a horizontal plane is measured by the orientation sensor 32. The orientation sensor 32 may be, for example, a geomagnetic sensor. The angle sensor 33 acquires an inclination with respect to a horizontal plane among pieces of posture information of the camera 20. An imaging angle, that is, an angle that a direction in which the camera 20 faces forms with a horizontal plane is measured by the angle sensor 33. The angle sensor 33 may be an acceleration sensor or an inclination sensor. Note that the measuring section 30 may include a sensor capable of measuring an orientation and an angle at the same time by combining the orientation sensor 32 and the angle sensor 33.

Display Part

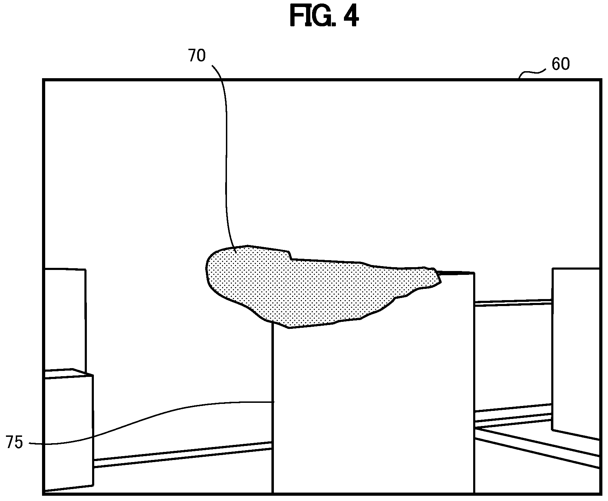

The display part 40 is a display device. The display part 40 displays images and information such as the operating status of each device. The display part 40 has a screen for displaying an image of a gas cloud. The screen of the display part 40 may be a touch screen that receives input from the user, and can receive operation and input to each device. As illustrated in Fig. 4, the user can visually recognize the gas cloud on the screen 60 of the display part 40. The user can adjust the position and direction of the camera 20 so that the gas cloud 70 is located at the center of the screen 60. There may be a plurality of display parts 40, which are added by a tablet terminal or the like.

Acquired Data Storage Section

The acquired data storage section 45 is a means for storing data. The acquired data storage section 45 stores the infrared image and the position information, the imaging orientation, and the imaging angle of the camera 20 in association with each other as acquired data. The position information, the imaging orientation, and the imaging angle have a small data amount. By associating the position information, the imaging orientation, and the imaging angle with the infrared image, the position information, the imaging orientation, and the imaging angle are used later in data processing such as flow rate estimation. The acquired data storage section 45 receives the red line image and the sensor information such as the position information, associates them with each other, and stores them in the storage device. The data after the association is the acquired data. The storage device of the acquired data storage section 45 may be a hard disk, a semiconductor memory, or the like.

Gas Flow Rate Estimation Device

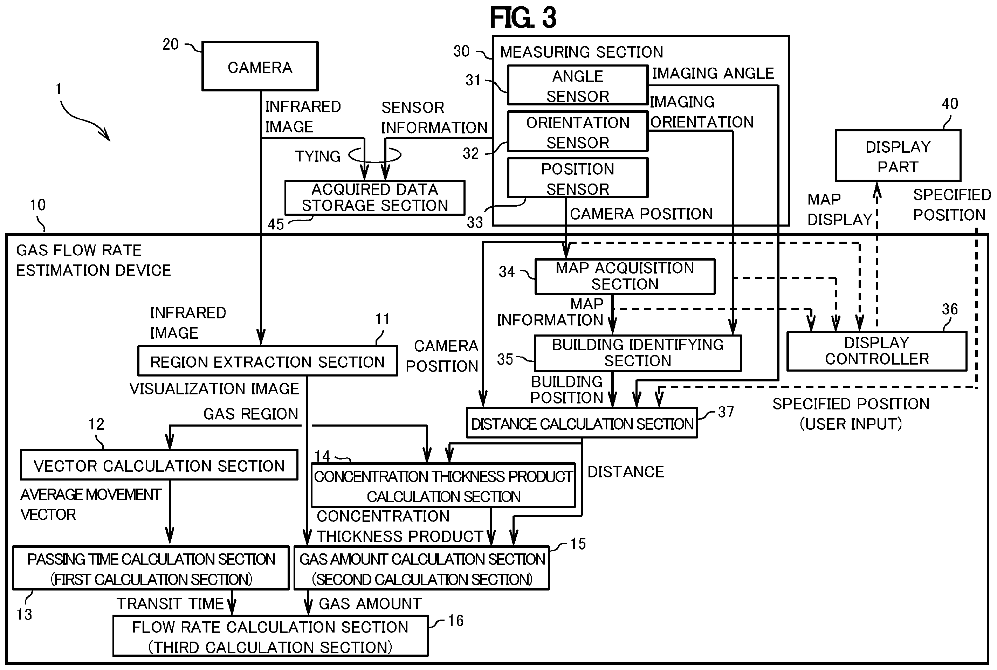

The gas flow rate estimation device 10 is an information processing apparatus. The gas flow rate estimation device 10 calculates the value of the gas flow rate from the image of the gas cloud. For example, the volume (L) of gas that passes through a cross section perpendicular to the screen in a unit time (min) is the gas flow rate (L/min). As illustrated in Fig. 3, the gas flow rate estimation device 10 includes a region extraction section 11, a vector calculation section 12, a transit time calculation section 13, a concentration thickness product calculation section 14, a gas amount calculation section 15, a flow rate calculation section 16, a map acquisition section 34, a building identifying section 35, and a distance calculation section 37. The passing time calculation section 13 is also referred to as a first calculation section. The gas amount calculation section 15 is also referred to as a second calculation section. The flow rate calculation section 16 is also referred to as a third calculation section.

Region Extraction Section

The region extraction section 11 is a means for visualizing a gas cloud from an infrared image and extracting a gas region. The gas region is a region in which pixels forming the visualized image of the gas cloud are distributed. The region extraction section 11 receives the time-series infrared images as input and outputs a visualization image and a gas region. As an example, the region extraction section 11 can perform the following first and second image processing. The first image processing visualizes an image of the gas cloud. From the time series of infrared images, temperature changes are observed. The frequency of the temperature change in the place where the gas cloud exists is higher than the frequency of the temperature change in the place where the gas cloud does not exist. The region extraction section 11 utilizes this fact to perform processing for removing low frequency components. For example, time-series image data of 30 frames per second is an infrared image. The region extraction section 11 takes a moving average of the luminance of each pixel in a predetermined number of frames such as 21 frames, for example, and calculates a difference from the infrared image. Even when the temperature change of the background is large, the gas cloud is detected by the first image processing. Note that the region extraction section 11 removes high-frequency components derived from noise or the like, and generates time-series visualization images which are images obtained by visualizing the gas clouds. Fig. 4 is an example of a screen on which a visualization image is superimposed and displayed on an infrared image. The first image processing is disclosed in, for example, Japanese Patent No. 6245418. Note that it is not necessary to rely on the technology disclosed therein.

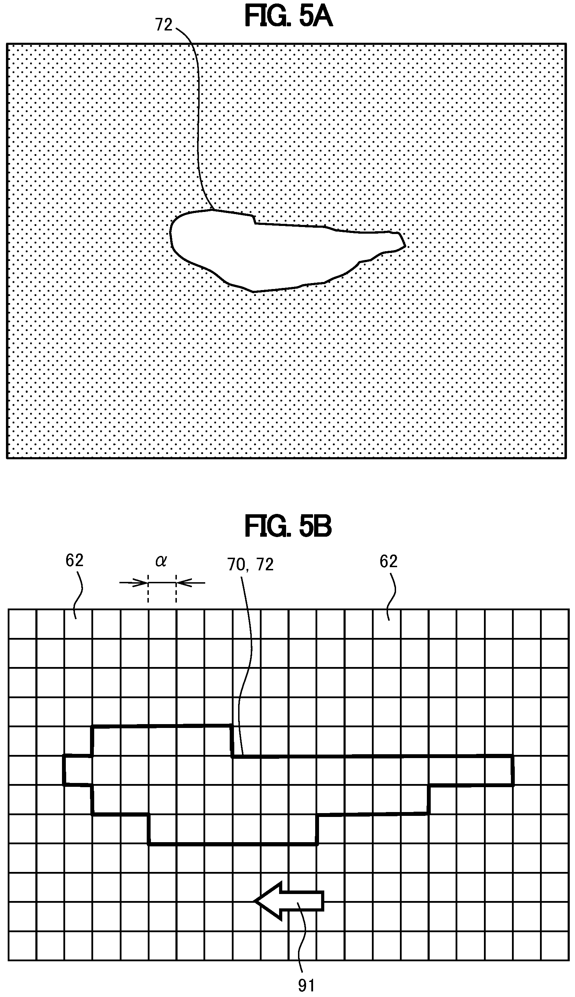

The second image processing extracts a gas region. The region extraction section 11 extracts, for example, the maximum value of the luminance of each pixel for each predetermined number of frames such as 30 frames from the time-series visualization images. Then, the region extraction section 11 generates an image in which the extracted maximum value is set to each pixel. The region extraction section 11 further performs noise removal and binarization processing to specify a gas region 72 as illustrated in Fig. 5A. The gas region 72 is time-series image data, and the gas region 72 is displayed to be superimposed on an infrared image or the like.

Vector Calculation Section

The vector calculation section 12 is a means for calculating an average movement vector of pixels. The motion vector represents the movement of an object or the like on the screen by a direction and a magnitude at the position of each pixel. The vector calculation section 12 receives the visualization image and the gas region as input and outputs an average movement vector of each pixel in the gas region. The vector calculation section 12 obtains a movement vector for each pixel corresponding to the gas region 72 with respect to the visualization image, and calculates an average. The movement vector is calculated by a known method such as template matching. Here, as illustrated in Fig. 5B, the average movement vector 91 of the gas region 72 is a vector in the longitudinal direction of the gas region 72. Note that the pixel 62 is illustrated in an enlarged manner in Fig. 5B. The actual number of pixels of the gas region 72 is, for example, a value such as 5032 pix. The average movement vector is, for example, a value such as 25.5 pix/sec. It is the average movement vector that represents that the gas is moving in the gas region 72.

Passing Time Calculation Section

The passing time calculation section 13 is a first calculation section. A means for estimating the time required for the gas to pass through the gas region 72 is the passing time calculation section 13. The passing time calculation section 13 receives the average movement vector and the gas region, and outputs a transit time. For example, the passing time calculation section 13 defines, on the screen, a rectangle that circumscribes the gas region 72 and has a side parallel to the average movement vector. Then, the gas passage time is calculated from the number of pixels corresponding to the length of the side of the rectangle and the number of pixels of the average movement vector. For example, when the number of pixels of a side of a rectangle is a value such as 172 pix and the average movement vector is 25.5 pix/sec, the transit time is 6.75 sec.

Concentration Thickness Product Calculation Section

The concentration thickness product calculation section 14 is means for calculating a concentration thickness product of a gas. The concentration thickness product of a gas is the product of the concentration of the gas and the thickness of the gas. The concentration thickness product calculation section 14 receives the visualization image and the gas region, and outputs the average value of the concentration thickness products of the pixels in the gas region. Further, as a parameter for calculating the concentration thickness product, there is distance information between the imaging position and the measurement target. The distance information is a distance in the depth direction. The gas flow rate estimation device 10 uses, as the value of the distance, a distance calculated by a distance calculation section to be described later. The concentration thickness product is disclosed in Japanese Patent No. 6344533. Note that it is not necessary to rely on the technology disclosed therein.

The gas cloud is swaying due to wind or the like. Therefore, even when the camera 20 is fixed, the intensity of the infrared ray detected by each pixel changes. Therefore, a change in the intensity of the infrared ray between when the gas exists and when the gas does not exist can be captured in some cases. For example, the concentration thickness product is calculated from the change in infrared intensity by previously determining the correlation between the ratio of infrared light absorbed by the gas and the concentration thickness product. The concentration thickness product calculation section 14 obtains the concentration thickness product of each pixel in the gas region and calculates the average thereof. The mean value of the concentration thickness product is, for example, a value such as 0.285%LELm. Note that the concentration of the gas is expressed as a ratio to the lower explosion limit (LEL) concentration. The volume concentration of the lower explosion limit is expressed as 100%LEL. The concentration thickness product is obtained by multiplying this by a depth direction as viewed from the camera 20, and is expressed in %LELm. The lowest concentration at which the combustible gas mixed with air causes explosion by ignition is a lower explosion limit. For example, in the case of methane, a volume concentration of 5% is 100%LEL and 0.285%LELm corresponds to 0.01425%m.

Gas Amount Calculation Section

The gas amount calculation section 15 is a second calculation section and is means for calculating the volume of gas. The gas amount calculation section 15 receives the concentration thickness product and the gas region as input and outputs the gas amount in the gas region. The gas amount calculation section 15 obtains the gas amount by multiplying the concentration thickness product by the area of the gas region. The area of the gas region is calculated by counting and multiplying the number of pixels of the gas region 72 as illustrated in Fig. 5B by the area corresponding to one pixel. The length corresponding to the width $\alpha$ of one pixel at the place of the gas cloud changes depending on the distance between the camera 20 and the gas cloud. The gas amount calculation section 15 calculates the gas amount using the distance calculated by the distance calculation section. The length corresponding to the width $\alpha$ of the pixel is, for example, a value such as 0.09219 m, and one pixel corresponds to the area of 0.008499 $m^2$. The area corresponding to the gas region where the number of pixels is 5032pix is 42.77 $m^2$. When the concentration thickness product is 0.0285%LELm (0.01425%m), the gas amount is calculated to be 6.095 L (0.006095 $m^3$).

Flow Rate Calculation Section

The flow rate calculation section 16 is a third calculation section and is means for calculating a gas flow rate. The flow rate calculation section 16 receives the gas amount and the gas passing time as inputs, and outputs a value obtained by dividing the gas amount by the gas passing time as a gas flow rate (L/min). For example, when the gas amount is 6.095 L and the transit time is 6.75 sec, the gas flow rate is calculated to be 54.2 L/min. The processing from the first image processing to the calculation of the gas flow rate is disclosed in, for example, Japanese Patent No. 6693609, except that the distance calculated by the distance calculation section is used. Note that it is not necessary to rely on the technology disclosed therein.

Map Acquisition Section

Next, processing for calculating the distance between the gas cloud and the camera 20 in the gas flow rate estimation device 10 is described. The calculation of the distance is performed by the map acquisition section 34, the building identifying section 35, and the distance calculation section 37. The map acquisition section 34 is a means for acquiring map information on the surroundings of the camera 20 from position information on the camera 20 that has captured the infrared image. The map acquisition section 34 receives position information of the camera 20 as input and outputs map information. The map information, if selectable, should be detailed information about the position of the building. Further, it is preferable to include the information of the outer peripheral line of the building. As the map information, map data prepared by a gas facility company or the like, a map information service provided through the Internet, or the like is used.

Building Identifying Section

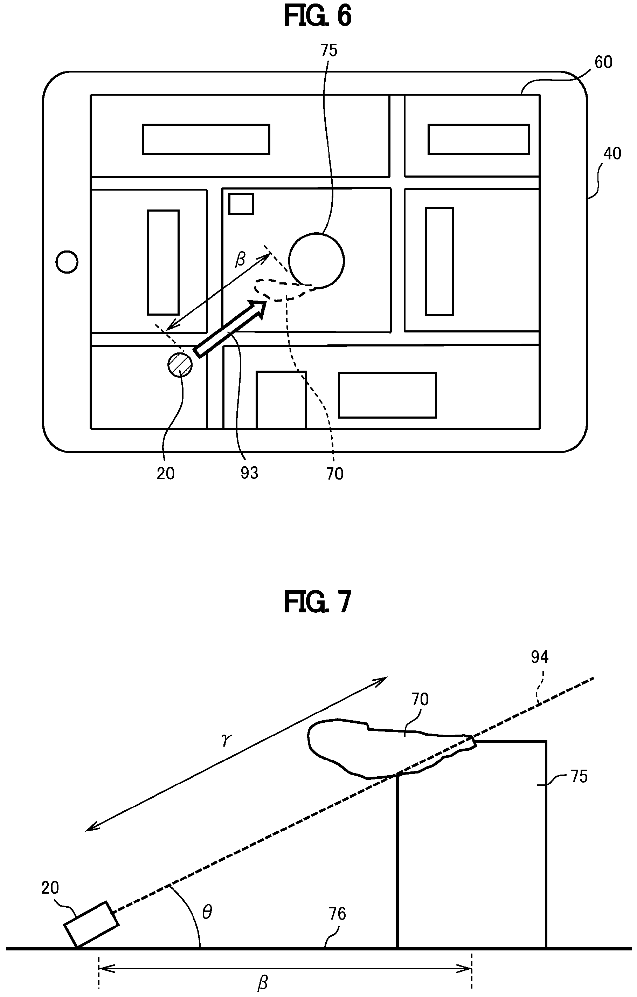

The building identifying section 35 is a means for identifying, on a map, a building to be imaged by the camera 20. The building identifying section 35 applies the imaging orientation 93 of the camera 20 that has captured the infrared image to the map information and identifies the nearest building 75 in the imaging orientation 93. The building identifying section 35 receives the position information on the camera 20 and the map information as input, and outputs the position information on the identified building, that is, the coordinate value. In a case where the information of the outer peripheral line of the building is included in the map information, the building identifying section 35 can output the position information of the outer peripheral line facing the camera 20. The building identifying section 35 automatically detects, from the map information, the nearest building located in the imaging orientation of the camera 20. Here, as illustrated in Fig. 6, a map is displayed on a screen 60 of the smartphone or the tablet terminal of the user, which is the display part 40, and the position of the camera 20 is displayed. The building identifying section 35 has identified the building 75 located nearest to the direction of the imaging orientation 93 of the camera 20. Note that no map may be displayed on the screen 60 when the building identifying section 35 automatically identifies a building, but a map can be displayed on the screen 60.

Distance Calculation Section

The distance calculation section 37 is a means for calculating the distance from the camera 20 to the gas cloud based on the map information. The distance calculation section 37 receives the position information of the camera 20 and the position information of the building or the position information of its outer peripheral line, and outputs the calculated distance. The distance calculation section 37 can calculate, for example, a horizontal distance and use the calculated distance as the distance from the camera 20 to the gas cloud. In the example of Fig. 6, the distance calculation section 37 calculates the plan view distance β from the camera 20 to the outer peripheral line of the building 75. The distance calculation section 37 can further receive the imaging angle of the camera 20 as an input and calculate and output a distance. As illustrated in Fig. 7, the distance calculation section 37 can calculate the three dimensional distance γ to the gas cloud 70 by trigonometry from the imaging angle θ of the camera 20 that has captured the infrared image and the plan view distance β between the building 75 and the camera 20. The three dimensional distance γ is a distance from the camera 20 to the gas cloud 70. Expression (1) for calculating the three dimensional distance γ by trigonometry is expressed as follows:

$$\gamma = \beta/\cos\theta \qquad (1)$$

Note that the angle formed by the direction 94 in which the camera 20 is facing and the horizontal plane is the imaging angle θ, but since the ground surface 76 is horizontal here, it is described as an angle from the ground surface.

The leakage gas detection device 1 having the above-described configuration includes a sensor that acquires position information, an imaging orientation, and an imaging angle. Then, the gas flow rate estimation device 10 acquires map information from the position information, calculates the distance from the camera to the gas cloud based on the map information, and calculates the gas flow rate using the distance. This eliminates the need for distance measurement as a separate operation from infrared image capturing, and user convenience is improved. As an important factor for determining accuracy of gas flow rate estimation, there is distance information to a target to be imaged. The leakage gas detection device 1 can automatically acquire accurate three dimensional distance information by associating the position information and the information of the imaging orientation and of the imaging angle which are acquired by the sensor of the measuring section 30 with the map information, and can perform simple and highly accurate flow rate estimation.

The leakage gas detection device 1 includes a map acquisition section that acquires map information, a building identifying section that identifies the nearest building in an imaging orientation, and a distance calculation section that calculates a distance from position information and an imaging angle. As a result, when the map information includes attribute information of a building or the like, an object that is first hit in the direction in which a camera faces is automatically recognized as a measurement target based on the attribute information and the imaging orientation. Furthermore, a distance to the actual position of an object to be imaged is acquired by calculation from the imaging angle, which further simplifies user's operation and further improves convenience. Conventionally, even when whether to calculate a gas flow rate is unknown, distance measurement and storage are required, which imposes a heavy burden on a user. It was impossible to perform flow rate estimation (flow rate estimation later) from data in which distance information is not stored. The leakage gas detection device 1 includes an acquired data storage section that stores, as acquired data, an infrared image in association with position information of, an imaging orientation of, and an imaging angle of the camera. As a result, a workload on a user at the time of imaging is reduced, and a flow rate can be estimated later.

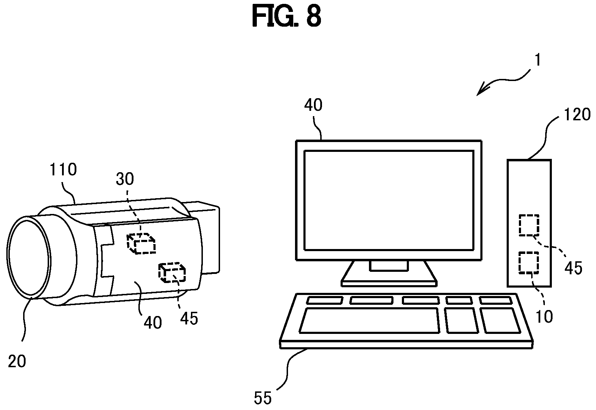

Note that as illustrated in Fig. 8, the camera 20 and the gas flow rate estimation device 10 may be separated from each other in the leakage gas detection device 1. Here, the camera 20 is a part of the imaging apparatus 110 together with the measuring section 30, and the gas flow rate estimation device 10 is incorporated in the computer 120. In addition, the leakage gas detection device 1 includes display parts 40 corresponding to the separate camera 20 and the separate gas flow rate estimation device 10 respectively. The imaging apparatus 110 and the computer 120 each have a communication interface and can communicate with each other. The imaging apparatus 110 may include the acquired data storage section 45, and the computer 120 may include the acquired data storage section 45. The leakage gas detection device 1 may include a plurality of imaging apparatuses 110 and a plurality of computers 120.

Modification Example

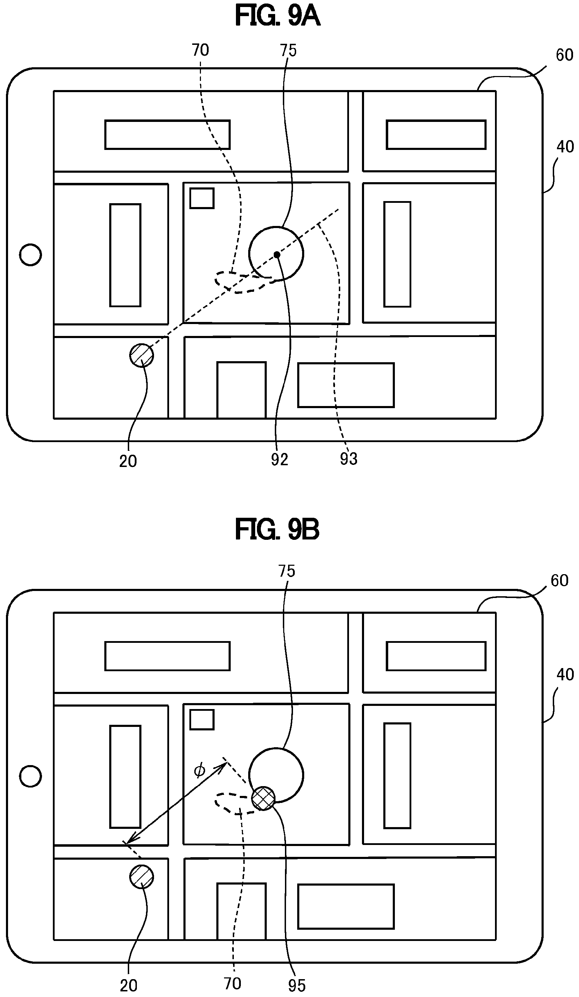

Next, a leakage gas detection device according to a modification example is described with reference to Fig. 9A and Fig. 9B. The leakage gas detection device according to the modification example further includes a display controller 36 that causes the screen 60 to display map information. Then, the distance calculation section 37 calculates a distance between the specified position 95 specified by a user in the map information and the camera 20. The calculated distance is defined as the distance from the camera 20 to the gas cloud 70. The other points are common to the leakage gas detection device 1 according to the embodiment.

Display Controller

The display controller 36 is means for displaying map information on the display part 40. The display controller 36 receives the map information, the position information of the camera 20, and the imaging orientation of the camera 20 as input, and outputs the map information including the position of the camera 20. As illustrated in Fig. 9A, the display controller 36 causes the position of the camera 20 to be displayed on the map displayed on the screen 60. Here, the display controller 36 positions the camera 20 at the edge of the screen. Then, the display controller 36 displays the map information so that the imaging orientation 93 of the camera 20 passes through the center 92 of the screen 60. As a result, the imaging orientation 93 of the camera 20 is widely displayed on the screen 60.

While viewing the screen 60, the user can designate the position of an object to be captured by the camera 20. As illustrated in Fig. 9B, here, the specified position 95 specified by the user is near the periphery of a building 75. The screen 60 may receive the input of the specified position 95 by the user as a touch screen. Further, the input of the specified position 95 by the user may be input by a mouse. Further, the input of the specified position 95 by the user may be an input of a numerical value of a coordinate by a keyboard or the like. Then, the distance calculation section 37 calculates the plan view distance $\varphi$ between the camera 20 and the specified position 95 specified by the user in the map information. The calculated distance is defined as a distance from the camera 20 to the gas cloud 70. Further, the distance calculation section 37 can calculate a three dimensional distance $\gamma$ by trigonometry from the position of the camera 20, the specified position 95, and the imaging angle $\theta$.

When the user designates a position of a target to be imaged on the map, the leakage gas detection device according to the modification example can reflect the correct position of the gas cloud in the acquired data, and the reliability of the acquired data is improved. Even in the case of map information in which information on buildings or the like is insufficient, the leakage gas detection device according to the modification example can calculate an accurate distance by the designation of the position by the user. In addition, even in a case in which the structure of the building is complicated, the leakage gas detection device according to the modification example can perform detailed designation of the position.

Detection Data Processing Device

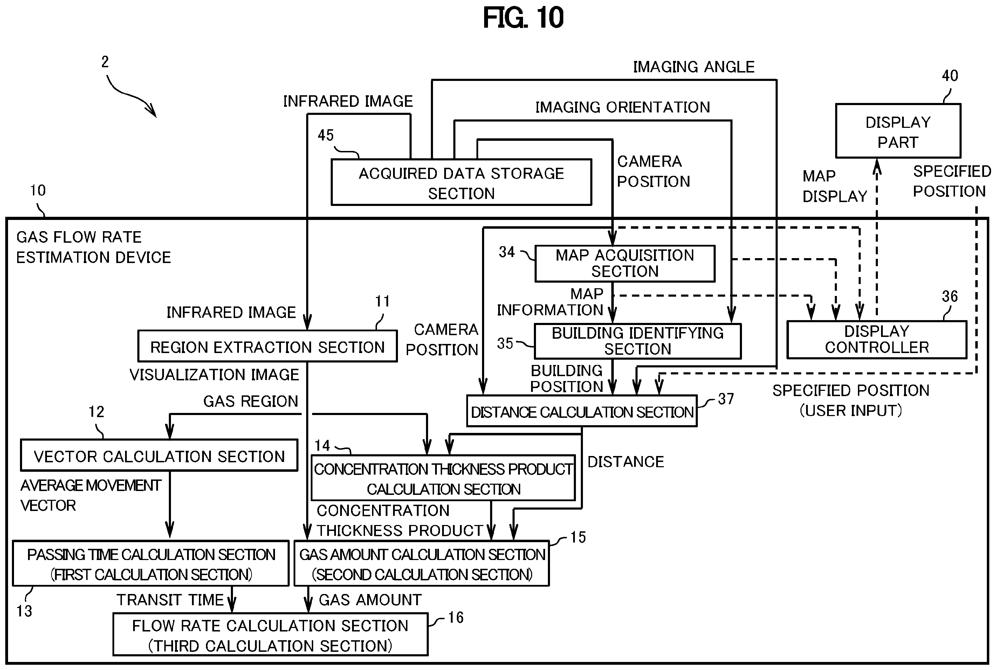

Next, the detection data processing device 2 according to an embodiment is described with reference to Fig. 10. The detection data processing device 2 is an information processing apparatus. The detection data processing device 2 calculates a gas flow rate from the acquired data that has already been acquired. As illustrated in Fig. 10, the detection data processing device 2 may not include the camera 20 and the measuring section 30. The detection data processing device 2 reads the acquired data and calculates a gas flow rate. Other points are common to the leakage gas detection device 1. The detection data processing device 2 includes a gas flow rate estimation device 10. The gas flow rate estimation device 10 reads acquired data in which an infrared image of a gas cloud is associated with position information of, an imaging orientation of, and an imaging angle of the camera 20 that has captured the infrared image, and estimates a gas flow rate. Here, the detection data processing device 2 reads the acquired data from the acquired data storage section 45. The acquired data storage section 45 may be an external storage device.

The detection data processing device 2 may further include a display part 40 having a screen for displaying an image of a gas cloud and a display controller 36 for displaying map information on the screen, similarly to the leakage gas detection device 1. Further, the display controller 36 can display the map information such that the imaging orientation of the camera 20 passes through the center of the screen.

Even in the case of the data of the infrared image in which the distance information is not stored, the detection data processing device 2 can estimate a flow rate later as long as there are the position information of, the imaging orientation of, and the imaging angle of the camera 20 associated with the infrared image. By the detection data processing device 2, a gas flow rate can be estimated with high accuracy as in the case of the leakage gas detection device 1, and a flow rate can be estimated later. Note that the detection data processing device 2 may include the camera 20 and the measuring section 30. For example, when acquired data is stored in the acquired data storage section 45, the leakage gas detection device 1 can be made to function as the detection data processing device 2. Furthermore, the leakage gas detection device 1 can also function as the detection data processing device 2 by connecting an external storage device in which the acquired data is stored to the leakage gas detection device 1. The computer 120 described as a case where the camera 20 and the gas flow rate estimation device 10 are separate bodies can also function as the detection data processing device 2.

Leakage Gas Detection Method

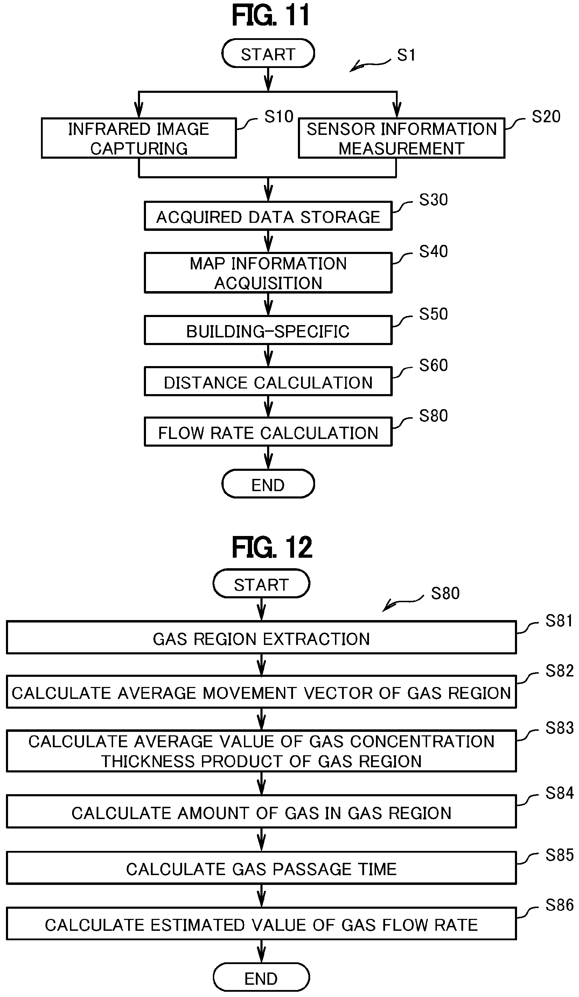

Next, referring to Figs. 11 to 13, the leakage gas detection methods S1 and S2 according to an embodiment is described. There are two leakage gas detection methods according to an embodiment, the leakage gas detection method S1 in which a building is automatically identified and the leakage gas detection method S2 in which a user specifies a position.

As illustrated in Fig. 11, the leakage gas detection method S1 for automatically identifying a building includes processing of the following steps S10 to S80. The flowchart of Fig. 11 is described below with reference to the respective parts of Figs. 3 and 10. First, the camera 20 captures the red line image of the gas cloud (step S10), and the measuring section 30 measures the sensor information including the position information of, the imaging orientation of, and the imaging angle of the camera 20 (step S20). The acquired data storage section 45 stores the infrared image captured by the camera 20 and the sensor information measured by the measuring section 30 in association with each other as acquired data (step S30). The map acquisition section 34 of the gas flow rate estimation device 10 acquires map information on the surroundings of the camera 20 from the position information on the camera 20 (step S40). The building identifying section 35 applies the imaging orientation of the camera 20 to the map information, and identifies the nearest building in the imaging orientation (step S50). The distance calculation section 37 calculates a plan view distance between the identified building and the camera 20 (step S60). In step S60, the distance calculation section 37 may calculate a three dimensional distance by trigonometry using the imaging angle. Next, when the gas flow rate estimation device 10 calculates a gas flow rate estimation value using the calculated distances (step S80), the processing S1 in Fig. 11 ends.

As illustrated in Fig. 12, the step S81 of calculating a flow rate includes the processing from the step S80 to the step S86 to be described next. The same applies to the step S80 of calculating a flow rate in the other flowcharts. Hereinafter, the flowchart of Fig. 12 is described with reference to the respective parts of Figs. 3 and 10. The region extraction section 11 extracts a gas region from time-series infrared images (step S81). Next, the vector calculation section 12 calculates an average movement vector of pixels on the basis of the gas regions extracted by the region extraction section 11 (step S82). The concentration thickness product calculation section 14 calculates the mean value of the gas concentration thickness products of the gas region (step S83). Using the time-series images of the gas cloud captured by the camera, the passing time calculation section 13, which is the first calculation section, calculates a gas velocity in the gas region and a gas passage time during which the gas passes through the gas region (step S85). The gas amount calculation section 15, which is a second calculation section, calculates the gas concentration thickness product of the gas region using the image data of the gas region included in the infrared image, and calculates the gas amount of the gas region using the calculated gas concentration thickness product and the distances to the gas clouds (step S84). The flow rate calculation section 16, which is a third calculation section, calculates a flow rate estimation value of the gas using the gas passage time and the gas amount (step S86). Note that after the processing of the step S82 in which the average movement vector is calculated, the processing of the step S86 in which the gas passage time is calculated can be performed before the step S85 in which the estimated value of the gas flow rate is calculated.

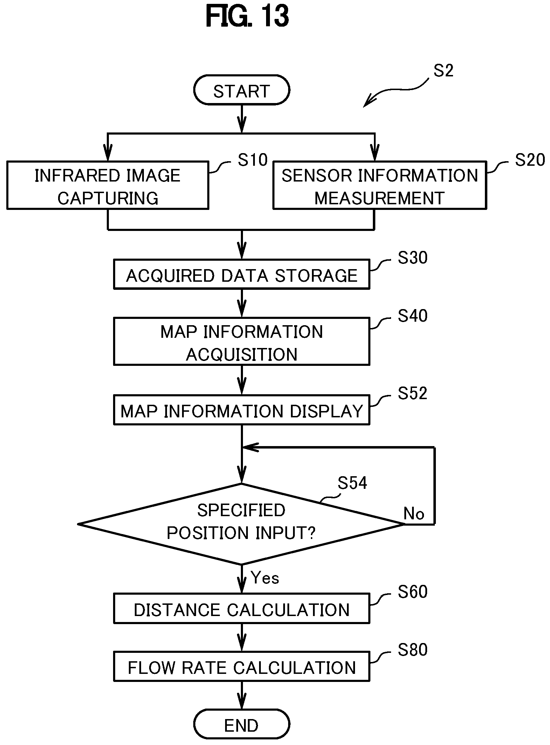

As illustrated in Fig. 13, in the leakage gas detection method S2 in which the user designates the position, steps S50 and S54 are executed instead of step S52. First, the camera 20 captures an infrared image of a gas cloud (step S10), and the measuring section 30 measures sensor information including position information of, an imaging orientation of, and an imaging angle of the camera 20 (step S20). The acquired data storage section 45 stores the infrared image captured by the camera 20 and the sensor information measured by the measuring section 30 in association with each other as acquired data (step S30). The map acquisition section 34 acquires map information on the surroundings of the camera 20 from the position information of the camera 20 (step S40). The display controller 36 of Fig. 10 displays the map information including the position of the camera 20 on the screen of the display part 40 (step S52). Thereafter, the distance calculation section 37 waits for an input of a specified position from the user, and receives the input of the specified position (step S54). Thereafter, the process proceeds to step S60. Then, the distance calculation section 37 calculates a distance between the position of the gas cloud specified by the user and the camera 20 (step S60). Then, the gas flow rate estimation device 10 calculates a gas flow rate estimation value using the calculated distance (step S80), and ends the process S2 of Fig. 13.

Note that the processing from the step S40 of acquiring map information to the step S80 of calculating a flow rate in the leakage gas detection method S1 of automatically identifying a building corresponds to the gas flow rate estimation method according to an embodiment. The processing from the step S40 of acquiring the map information to the step S80 of calculating a flow rate in the leakage gas detection method S2 in which a user specifies a position corresponds to the gas flow rate estimation method according to the embodiment.

The leakage gas detection methods S1 and S2 are each divided into two processes, i.e., a data acquisition process and a data reading process. In the data acquisition process, the leakage gas detection device 1 captures an infrared image and measures sensor information, and stores them in association with each other as acquired data. In the data reading process, the leakage gas detection device 1 reads the stored acquired data, calculates a distance between the camera and the imaging target, and calculates a gas flow rate using the distance.

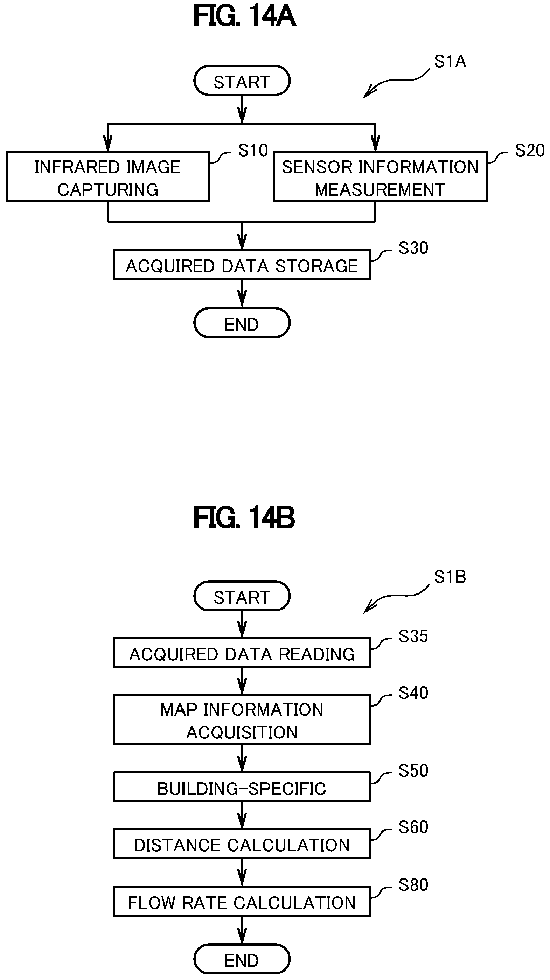

Fig. 14A is a flowchart of the data acquisition process S1A in the leakage gas detection method S1 for automatically identifying a building. First, the camera 20 captures an infrared image (step S10), and the measuring section 30 measures sensor information (step S20). Thereafter, when the acquired data storage section 45 stores the infrared image and the sensor information in association with each other as the acquired data (step S30), the process S1A of Fig. 14A is ended.

Fig. 14B is a flowchart of the data reading process S 1B of the leakage gas detection method S1 for automatically identifying a building. First, the acquired data storage section 45 reads the acquired data in which an infrared image and sensor information are associated with each other (step S35). The sensor information includes position information of, an imaging orientation of, and an imaging angle of the camera. The map acquisition section 34 of the gas flow rate estimation device 10 acquires map information around the camera from the position information of the camera (step S40). The building identifying section 35 applies the imaging orientation of the camera to the map information and identifies the nearest building in the imaging orientation (step S50). The distance calculation section 37 calculates a plan view distance between the identified building and the camera (step S60). Next, when the gas flow rate estimation device 10 calculates a gas flow rate estimation value by using the calculated distance (step S80), the processing of Fig. 14B ends.

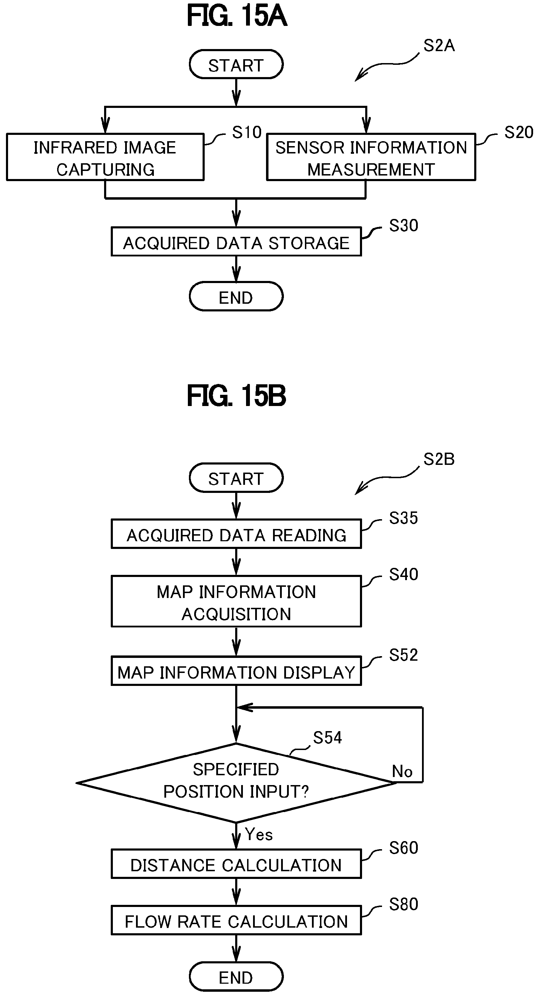

The data acquisition process S2A of the leakage gas detection method S2 in which a user specifies a position is shown in Fig. 15A. In this process S2A, the same processing as steps S10 to S30 of Fig. 14A is performed. Fig. 15B is a flowchart of the gas flow rate calculation process S2B in the leakage gas detection method S2 in which a position is designated by a user.

First, the acquired data storage section 45 reads the acquired data in which an infrared image and sensor information are associated with each other (step S35). The sensor information includes position information of, an imaging orientation of, and an imaging angle of the camera. The map acquisition section 34 of the gas flow rate estimation device 10 acquires map information around the camera from the position information of the camera (step S40). The display controller 36 of Fig. 10 displays the map information including the position of the camera on the screen (step S52). Thereafter, the distance calculation section 37 waits for an input of a specified position from the user, and receives the input of the specified position (step S54). Thereafter, the process proceeds to step S60. Next, the distance calculation section 37 calculates a distance between the position specified by the user and the camera (step S60). Then, the gas flow rate estimation device 10 calculates a gas flow rate by using the calculated distance (step S80), and ends the process of Fig. 15B. The leakage gas detection device 1 can independently perform the data acquisition processes S1A and S2A and the data reading processes S1B and S2B.

Gas Flow Rate Estimation Program

A gas flow rate estimation program according to an embodiment is a program that is read by a computer and causes the computer to execute processing of a gas flow rate estimation method. The gas flow rate estimation program is acquired through an electric communication line and recorded in a computer-readable recording medium.

The leakage gas detection device, the leakage gas detection method, and the like according to embodiments are capable of estimating a flow rate with high accuracy while eliminating the need for distance measurement by a user at the time of capturing an infrared image.

Although embodiments of the present invention have been described and illustrated in detail, the disclosed embodiments are made for purposes of illustration and example only and not limitation. The scope of the present invention should be interpreted by terms of the appended claims.

**Claims**

**1.** A gas flow rate estimation device (10) comprising:

a map acquisition section (34) configured to acquire, from position information of a camera (20) capturing an image including information of an infrared ray, map information around the camera (20);
a distance calculation section (37) configured to calculate, based on the map information, a distance from the camera (20) to a gas cloud captured by the camera (20);
a first calculation section (13) configured to calculate, by using time-series images of the gas cloud captured by the camera (20), a gas velocity of the gas cloud and a gas passage time for which gas passes through a gas region extracted from the time-series images;
a second calculation section (15) configured to calculate a gas concentration thickness product of the gas region by using image data of the gas region included in the time-series images and configured to calculate a gas amount of the gas region by using the gas concentration thickness product and a distance calculated by the distance calculation section (37); and
a third calculation section (16) configured to calculate a flow rate estimation value of gas by using the gas passage time and the gas amount.

**2.** The gas flow rate estimation device (10) according to claim 1, further comprising a building identifying section (35) configured to apply an imaging orientation of the camera (20) to the map information and configured to identify a nearest building in the imaging orientation, and wherein
the distance calculation section (37) is configured to calculate a distance between a building identified by the building identifying section (35) and the camera (20) and is configured to use the distance as a distance from the camera (20) to the gas cloud.

3. The gas flow rate estimation device (10) according to claim 2, wherein
the distance calculation section (37) is configured to calculate a three dimensional distance from the camera (20) to the gas cloud by using trigonometry based on an imaging angle of the camera (20) and a plan view distance between the building and the camera (20).

4. The gas flow rate estimation device (10) according to claim 1, wherein
the distance calculation section (37) is configured to calculate a distance from the camera (20) to the gas cloud with a specified position specified by a user in the map information as a position of the gas cloud.

5. The gas flow rate estimation device (10) according to claim 4, wherein
the distance calculation section (37) is configured to calculate a three dimensional distance from the camera (20) to the gas cloud by trigonometry based on an imaging angle of the camera (20) and a plan view distance between the specified position and the camera (20).

6. A leakage gas detection device (1) comprising:

   the camera (20);
   a measuring section (30) including:

      a position sensor (31) configured to acquire position information of the camera (20);
      an orientation sensor (32) configured to detect an imaging orientation of the camera (20); and
      an angle sensor (33) configured to detect an imaging angle of the camera (20);

   a display part (40) including a screen (60) configured to display an image of the gas cloud; and
   the gas flow rate estimation device (10) according to any one of claims 1 to 5.

7. The leakage gas detection device (1) according to claim 6, further comprising an acquired data storage section (45) configured to store, as acquired data, the image in association with position information of, an imaging orientation of, and an imaging angle of the camera (20).

8. The leakage gas detection device (1) according to claim 6, further comprising a display controller (36) configured to cause the map information to be displayed on the screen (60).

9. The leakage gas detection device (1) according to claim 8, wherein
the display controller (36) is configured to cause the map information to be displayed such that an imaging orientation of the camera (20) passes through a center (92) of the screen (60).

10. A detection data processing device (2) comprising the gas flow rate estimation device (10) according to any one of claims 1 to 5, wherein
the gas flow rate estimation device (10) is configured to estimate a gas flow rate by reading acquired data in which an infrared image obtained by imaging the gas cloud is associated with position information of, an imaging orientation of, and an imaging angle of a camera (20) capturing the infrared image.

11. The detection data processing device (2) according to claim 10, further comprising:

    a display part (40) including a screen (60) configured to display an image of the gas cloud; and
    a display controller (36) configured to cause the map information to be displayed on the screen (60).

12. The detection data processing device (2) according to claim 11, wherein
the display controller (36) is configured to cause the map information to be displayed such that an imaging orientation of the camera (20) passes through a center (92) of the screen (60).

13. A gas flow rate estimation method comprising:

    acquiring map information around a camera (20) capturing an infrared image of a gas cloud from position information of the camera (20);
    calculating a distance from the camera (20) to the gas cloud based on the map information;
    calculating, by using time-series images of the gas cloud captured by the camera (20), a gas velocity of the gas cloud and a gas passage time for which gas passes through a gas region extracted from the time-series images;
    calculating a gas concentration thickness product of the gas region by using image data of the gas region included in the infrared image and a gas amount of the gas region by using the gas concentration thickness product and a distance to the gas cloud; and
    calculating a flow rate estimation value of gas by using the gas passage time and the gas amount.

**14.** A gas flow rate estimation program causing a computer to perform:

acquiring map information around a camera (20) capturing an infrared image of a gas cloud from position information of the camera (20); calculating a distance from the camera (20) to the gas cloud based on the map information; calculating, by using time-series images of the gas cloud captured by the camera (20), a gas velocity of the gas cloud and a gas passage time for which gas passes through a gas region extracted from the time-series images; calculating a gas concentration thickness product of the gas region by using image data of the gas region included in the time-series images and a gas amount of the gas region by using the gas concentration thickness product and a distance to the gas cloud; and calculating a flow rate estimation value of gas by using the gas passage time and the gas amount.

# FIG. 1

# FIG. 2

## FIG. 3

CAMERA **20**

INFRARED IMAGE

SENSOR INFORMATION

**30** MEASURING SECTION

**31** ANGLE SENSOR — IMAGING ANGLE

**32** ORIENTATION SENSOR — IMAGING ORIENTATION

**33** POSITION SENSOR — CAMERA POSITION

TYING

ACQUIRED DATA STORAGE SECTION **45**

**40** DISPLAY PART

MAP DISPLAY

SPECIFIED POSITION

**10** GAS FLOW RATE ESTIMATION DEVICE

INFRARED IMAGE

**11** REGION EXTRACTION SECTION

VISUALIZATION IMAGE

GAS REGION

**34** MAP ACQUISITION SECTION

MAP INFORMATION

CAMERA POSITION

**35** BUILDING IDENTIFYING SECTION

BUILDING POSITION

**36** DISPLAY CONTROLLER

SPECIFIED POSITION (USER INPUT)

**37** DISTANCE CALCULATION SECTION

**12** VECTOR CALCULATION SECTION

AVERAGE MOVEMENT VECTOR

**14** CONCENTRATION THICKNESS PRODUCT CALCULATION SECTION — DISTANCE

CONCENTRATION THICKNESS PRODUCT

**13** PASSING TIME CALCULATION SECTION (FIRST CALCULATION SECTION)

**15** GAS AMOUNT CALCULATION SECTION (SECOND CALCULATION SECTION)

TRANSIT TIME

GAS AMOUNT

**16** FLOW RATE CALCULATION SECTION (THIRD CALCULATION SECTION)

EP 4 667 888 A1

14

## FIG. 4

## FIG. 5A

## FIG. 5B

## FIG. 6

## FIG. 7

# FIG. 8

## FIG. 9A

## FIG. 9B

## FIG. 10

## FIG. 11

START — S1

INFRARED IMAGE CAPTURING — S10    SENSOR INFORMATION MEASUREMENT — S20

ACQUIRED DATA STORAGE — S30

MAP INFORMATION ACQUISITION — S40

BUILDING-SPECIFIC — S50

DISTANCE CALCULATION — S60

FLOW RATE CALCULATION — S80

END

## FIG. 12

START — S80

GAS REGION EXTRACTION — S81

CALCULATE AVERAGE MOVEMENT VECTOR OF GAS REGION — S82

CALCULATE AVERAGE VALUE OF GAS CONCENTRATION THICKNESS PRODUCT OF GAS REGION — S83

CALCULATE AMOUNT OF GAS IN GAS REGION — S84

CALCULATE GAS PASSAGE TIME — S85

CALCULATE ESTIMATED VALUE OF GAS FLOW RATE — S86

END

# FIG. 13

S2

START

INFRARED IMAGE CAPTURING — S10

SENSOR INFORMATION MEASUREMENT — S20

ACQUIRED DATA STORAGE — S30

MAP INFORMATION ACQUISITION — S40

MAP INFORMATION DISPLAY — S52

SPECIFIED POSITION INPUT? — S54    No

Yes

DISTANCE CALCULATION — S60

FLOW RATE CALCULATION — S80

END

## FIG. 14A

```
        ( START )                    ⟋ S1A
            │
    ┌───────┴────────┐
    ▼                ▼
┌──────────────┐  ┌──────────────────┐
│ INFRARED     │  │ SENSOR INFORMATION│── S20
│ IMAGE        │──│ MEASUREMENT      │
│ CAPTURING    │  └──────────────────┘
└──────────────┘── S10
    │                │
    └───────┬────────┘
            ▼
┌──────────────────────┐
│ ACQUIRED DATA STORAGE│── S30
└──────────────────────┘
            │
            ▼
         ( END )
```

## FIG. 14B

```
        ( START )                    ⟋ S1B
            │
            ▼
┌──────────────────────┐
│ ACQUIRED DATA READING│── S35
└──────────────────────┘
            │
            ▼
┌──────────────────────┐
│ MAP INFORMATION      │── S40
│ ACQUISITION          │
└──────────────────────┘
            │
            ▼
┌──────────────────────┐
│ BUILDING-SPECIFIC    │── S50
└──────────────────────┘
            │
            ▼
┌──────────────────────┐
│ DISTANCE CALCULATION │── S60
└──────────────────────┘
            │
            ▼
┌──────────────────────┐
│ FLOW RATE CALCULATION│── S80
└──────────────────────┘
            │
            ▼
         ( END )
```

# FIG. 15A

S2A

START

INFRARED IMAGE CAPTURING — S10    SENSOR INFORMATION MEASUREMENT — S20

ACQUIRED DATA STORAGE — S30

END

# FIG. 15B

S2B

START

ACQUIRED DATA READING — S35

MAP INFORMATION ACQUISITION — S40

MAP INFORMATION DISPLAY — S52

SPECIFIED POSITION INPUT? — S54    No

Yes

DISTANCE CALCULATION — S60

FLOW RATE CALCULATION — S80

END

| | Europäisches Patentamt European Patent Office Office européen des brevets | **EUROPEAN SEARCH REPORT** | **Application Number** EP 25 18 1798 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2020/110411 A1 (KONICA MINOLTA INC [JP]) 4 June 2020 (2020-06-04) * the whole document * * figures 3-10, 18 * ----- | 1-14 | INV. G01M3/38 G01N21/3504 |
| Y | US 2021/264584 A1 (ZHANG XIAOCHEN [JP] ET AL) 26 August 2021 (2021-08-26) * abstract; claims; figures * * paragraphs [0001] - [0004], [0031] - [0082] * ----- | 1-14 | |
| A,D | JP 6 693609 B1 (KONICA MINOLTA INC.) 13 May 2020 (2020-05-13) * the whole document * ----- | 1-14 | |
| A | EP 3 392 635 A1 (KONICA MINOLTA INC [JP]) 24 October 2018 (2018-10-24) * the whole document * ----- | 1-14 | |

| TECHNICAL FIELDS SEARCHED (IPC) |
|---|
| G01M G01N |

The present search report has been drawn up for all claims

| Place of search | Date of completion of the search | Examiner |
|---|---|---|
| The Hague | 6 November 2025 | Stavroulis, Stefanos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 1798

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2025

| Patent document cited in search report | Publication date | Patent family member(s) | Publication date |
|---|---|---|---|
| WO 2020110411 A1 | 04-06-2020 | NONE | |
| US 2021264584 A1 | 26-08-2021 | JP WO2020008688 A1 | 30-09-2021 |
| | | US 2021264584 A1 | 26-08-2021 |
| | | WO 2020008688 A1 | 09-01-2020 |
| JP 6693609 B1 | 13-05-2020 | JP 6693609 B1 | 13-05-2020 |
| | | JP WO2020110411 A1 | 15-02-2021 |
| | | US 2022034742 A1 | 03-02-2022 |
| EP 3392635 A1 | 24-10-2018 | EP 3392635 A1 | 24-10-2018 |
| | | JP 6319527 B2 | 09-05-2018 |
| | | JP WO2017104617 A1 | 01-03-2018 |
| | | US 2019003919 A1 | 03-01-2019 |
| | | WO 2017104617 A1 | 22-06-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020110411 A
- JP 6245418 B
- JP 6344533 B
- JP 6693609 B